# EUROPEAN PATENT SPECIFICATION

(11) **EP 4 320 403 B1**
(45) Date of publication and mention of the grant of the patent: **21.10.2026**
(21) Application number: 22725326.7
(22) Date of filing: 14.04.2022
(51) Int. Cl.: G01B 11/16, G01D 5/353

(54) **EXTENDED FIBER OPTIC SENSING SYSTEM**
ERWEITERTES FASEROPTISCHES SENSORSYSTEM
SYSTÈME DE DÉTECTION À FIBRE OPTIQUE ÉTENDUE

(30) Priority: 15.04.2021 US 202163175449 P
(43) Date of publication of application: 14.02.2024
(73) Proprietor: Bard Access Systems, Inc., Salt Lake City, UT 84116 (US)
(72) Inventor: SOWARDS, Steffan, Salt Lake City, UT 84124 (US); MISENER, Anthony K., Bountiful, UT 84010 (US); MCLAUGHLIN, William Robert, Bountiful, UT 84010 (US)
(74) Representative: Hoffmann Eitle
(86) International application number: PCT/US2022/024934
(87) International publication number: WO 2022/221608

(56) References cited:
- EP-A1- 3 560 417
- WO-A1-2017/203271
- US-A1- 2017 052 091
- US-A1- 2020 188 036

## Description

### PRIORITY

This application claims the benefit of priority to U.S. Provisional Application No. 63/175,449, filed April 15, 2021, which is incorporated by reference in its entirety into this application.

### BACKGROUND

In the past, certain intravascular guidance of medical instruments, such as guidewires and catheters for example, have used fluoroscopic methods for tracking tips of the medical instruments and determining whether distal tips are appropriately localized in their target anatomical structures. However, such fluoroscopic methods expose patients and their attending clinicians to harmful X-ray radiation. Moreover, in some cases, the patients are exposed to potentially harmful contrast media needed for the fluoroscopic methods.

More recently, electromagnetic tracking systems have been used involving stylets. Generally, electromagnetic tracking systems feature three components: a field generator, a sensor unit and control unit. The field generator uses several coils to generate a position-varying magnetic field, which is used to establish a coordinate space. Attached to the stylet, such as near a distal end (tip) of the stylet for example, the sensor unit includes small coils in which current is induced via the magnetic field. Based on the electrical properties of each coil, the position and orientation of the medical instrument may be determined within the coordinate space. The control unit controls the field generator and captures data from the sensor unit.

Although electromagnetic tracking systems avoid line-of-sight reliance in tracking the tip of a stylet while obviating radiation exposure and potentially harmful contrast media associated with fluoroscopic methods, electromagnetic tracking systems are prone to interference. More specifically, since electromagnetic tracking systems depend on the measurement of magnetic fields produced by the field generator, these systems are subject to electromagnetic field interference, which may be caused by the presence of many different types of consumer electronics such as cellular telephones. Additionally, electromagnetic tracking systems are subject to signal drop out, depend on an external sensor, and are defined to a limited depth range.

In some instances, fiber optic shape sensing technology may be used to facilitate tracking of a medical instrument. Fiber optic 3D shape sensing involves localizing and quantifying deformation occurring at one or more locations along the length of a sensing region of an optical fiber. Such deformation may be a complex combination of bending, twisting, and axial elongation. In some instances, multiple reflective fiber Bragg gratings disposed along a sensing region may define reflected light characteristics consisting with the shape of the elongate device along the sensing region. An optical fiber may be defined to operate across a defined spectrum of light which in some instances may be limited. The spectrum is divided into spectral widths assigned to each grating along the optical fiber such that each grating operates across its spectral width.

Optical shape sensing via multiple fiber Bragg gratings disposed along a lengthy sensing region of an optical fiber (consistent with an elongate medical device) may suffer from competing operational characteristics. On one side, a high number of gratings along a sensing region may require the limited light spectrum to be divided up into narrow spectral widths thus limiting the degree of wavelength shift afforded to the grating. On the other side, a lower number of gratings increases the spacing distance between gratings where the instrument strain cannot be determined. As such there is a need to optically sense the shape of an elongate instrument without limiting the size of the spectral width and without increasing the spacing between gratings.
US 2020/188036 A1 relates to a fiber optic tracking sensor including at least three optical fibers, each optical fiber having a plurality of fiber optic sensors along a length of a sensing portion of the sensor. A further prior art fiber optic device is known from EP 3 560 417.

### SUMMARY

The present invention is defined by the elongate multi-core optical fiber instrument according to independent claim 1 and the medical system according to independent claim 12. Other preferred optional features are recited in the dependent claims. Briefly summarized, disclosed herein is an elongate multi-core optical fiber instrument for insertion within a patient body. The multi-core optical fiber instrument includes a set of first optical fiber cores extending along a first sensing region of the multi-core optical fiber instrument, where each first optical fiber core includes a set of first sensors disposed along the first region. The multi-core optical fiber instrument further includes a set of second optical fiber cores extending along a second sensing region of the multi-core optical fiber instrument, where each second optical fiber core includes a set of second sensors disposed along the second sensing region. The first sensing region is located distal the second sensing region, and the first optical fiber cores extend along the second sensing region.

The set of first sensors is configured to operate across a set of first spectral widths of a light spectrum such that each first sensor of one first fiber core operates across a different spectral width from the other first sensors of the one first fiber core, and the set of second sensors is configured to operate across a set of second spectral widths of the light spectrum such that each second sensor of one second fiber core operates across a different spectral width from the other second sensors of the one second fiber core.

In some embodiments, the set of first spectral widths are the same for each first fiber core, and the set of second spectral widths are the same for each second fiber core. In some embodiments, the second spectral widths are a subset of the first spectral widths. In other embodiments, a majority of the set of second spectral widths are a subset of the first spectral widths and in still other embodiments, a substantial entirety of the second spectral widths are a subset of the first spectral widths.

The first sensors may be configured to determine one or more physical parameters of the multi-core optical fiber instrument along the first sensing region, and the second sensors may also be configured to determine the one or more physical parameters of the multi-core optical fiber instrument along the second sensing region. The physical parameters may include at least one of a bending direction, a shape, a torsional strain, a longitudinal strain, and a motion of the instrument.

The multi-core optical fiber instrument may define a circular cross section along the first region and one or more of the first optical fiber cores may be disposed adjacent a circumferential surface of the multi-core optical fiber instrument along the first region. In some embodiments, at least three of the first optical fiber cores are disposed adjacent the circumferential surface. The at least three first optical fiber cores may be equally spaced along the circumference surface. The at least three first optical fiber cores may extend along the first region at a constant angular position with respect to the multi-core optical fiber instrument. In some embodiments, the first sensors are equally spaced along the first sensing region defining a first separation distance.

The multi-core optical fiber instrument defines a circular cross section along the second sensing region, and one or more of the second optical fiber cores are disposed adjacent a circumferential surface of the multi-core optical fiber instrument along the second region. At least three of the second optical fiber cores are disposed adj acent the circumferential surface of the second region. The at least three second optical fiber cores may be equally spaced along the circumference surface of the second region and the at least three second optical fiber cores may extend along the second sensing region at a constant angular position with respect to the multi-core optical fiber instrument. At least one of the at least three second optical fiber cores is disposed at the same angular position as one of the at least three first optical fiber cores. In some embodiments, each of the at least three second optical fiber cores is disposed at the same angular position as a separate one of the at least three first optical fiber cores. The second sensors may be equally spaced along the second sensing region defining a second separation distance and the second separation distance may be equal to the first separation distance. In some embodiments, the second sensing region at least partially overlaps the first sensing region. In some embodiments, at least one of the first optical fiber cores is disposed adjacent the circumferential surface along the second sensing region.

The multi-core optical fiber instrument may be incorporated into a medical device such as an introducer wire, a guidewire, a stylet, a probe, a sheath, a mesh, a tube, an obturator, or a catheter.

Also disclosed herein is a system for sensing one or more parameters experienced by an elongate medical device inserted within a patient body. The system includes an elongate multi-core optical fiber instrument and the elongate multi-core optical fiber instrument includes: (i) a set of first optical fiber cores extending along a first sensing region of the multi-core optical fiber instrument, where each first optical fiber core includes a set of first sensors disposed along the first sensing region, and (ii) a set of second optical fiber cores extending along a second sensing region of the multi-core optical fiber instrument, where each second optical fiber core includes a set of second sensors disposed along the second sensing region.

The system further includes a console including one or more processors and a non-transitory computer-readable medium having logic stored thereon. The logic when executed by the one or more processors, causes operations. The operations include providing an incident light signal to the multi-core optical fiber instrument, receiving reflected light signals from the first sensors across a set of first spectral widths, and receiving reflected light signals from the second sensors across a set of second spectral widths. In some embodiments, the second spectral widths are a subset of the first spectral widths.

The operations further include processing the reflected light signals associated with the first sensors and the second sensors so as to determine a shape of the multi-core optical fiber instrument along the first sensing region from reflected light associated with the first sensors and a shape of the multi-core optical fiber instrument along the second sensing region from reflected light associated with the second sensors. The operations also include causing a rendering on a graphical display of the shape of the multi-core optical fiber instrument along the first sensing region and the second sensing region.

The multi-core optical fiber instrument may further include a set of third optical fiber cores extending along a length of the multi-core optical fiber instrument, where each third optical fiber core comprising one or more third sensors disposed along the length of the multi-core optical fiber instrument. Each sensor is configured to determine a physiological parameter of the patient body, and the physiological parameter is one of a body temperature, a fluid flow rate, or a pulse oximetry.

In some embodiments, the multi-core optical fiber instrument is incorporated into one of an introducer wire, a guidewire, a stylet, a probe, a sheath, a mesh, a tube, an obturator, or a catheter.

Also disclosed herein is a medical system for sensing one or more parameters experienced by an elongate medical device inserted within a patient body. The system includes an elongate multi-core optical fiber instrument incorporated into the medical device, where the medical device is one of an introducer wire, a guidewire, a stylet, a probe, a sheath, a mesh, a tube, an obturator, or a catheter.

The multi-core optical fiber instrument includes a set of first optical fiber cores extending along a first sensing region of the multi-core optical fiber instrument, where each first optical fiber core includes a set of first sensors disposed along the first sensing region. The multi-core optical fiber instrument further includes a set of second optical fiber cores extending along a second sensing region of the multi-core optical fiber instrument, where each second optical fiber core includes a set of second sensors disposed along the second sensing region.

The system further includes a console including one or more processors and a non-transitory computer-readable medium having stored thereon logic that, when executed by the one or more processors, causes operations of the system. The operations include: (i) providing an incident light signal to the multi-core optical fiber instrument, (ii) receiving reflected light signals from the first sensors across a set of first spectral widths, and (iii) receiving reflected light signals from the second sensors across a set of second spectral widths, where the second spectral widths are a subset of the first spectral widths.

The operations further include: (i) processing the reflected light signals associated with the first sensors and the second sensors, (ii) determining a shape of the multi-core optical fiber instrument along the first sensing region from reflected light associated with the first sensors, (iii) determining a shape of the multi-core optical fiber instrument along the second sensing region from reflected light associated with the second sensors, and (iv) causing a rendering on a graphical display of the shape of the multi-core optical fiber instrument along the first sensing region and the second sensing region.

In some embodiments, the multi-core optical fiber instrument further includes one or more third optical fiber cores extending along the multi-core optical fiber instrument, where each third optical fiber core includes at least one third sensor. In such an embodiment, the logic, when executed by the one or more processors, further causes operations including: (i) receiving reflected light signals from the at least one third sensor across a first spectral width, (ii) determining a physiological parameter of the patient body from reflected light associated with the at least one third sensor, and (iii) causing a rendering on the graphical display depicting the physiological parameter along the multi-core optical fiber instrument in combination with the shape of the multi-core optical fiber instrument along the first sensing region and the second sensing region. The physiological parameter may be one of a body temperature, a blood flow rate, or a pulse oximetry.

These and other features of the concepts provided herein will become more apparent to those of skill in the art in view of the accompanying drawings and following description, which disclose particular embodiments of such concepts in greater detail.

### BRIEF DESCRIPTION OF THE DRAWINGS

Embodiments of the disclosure are illustrated by way of example and not by way of limitation in the figures of the accompanying drawings, in which like references indicate similar elements and in which:
FIG. 1A is an illustrative embodiment of a multi-core optical fiber instrument with optical sensing capabilities, in accordance with some embodiments;
FIG. 1B illustrates cross section of a first sensing region of the multi-core optical fiber instrument of FIG. 1A, in accordance with some embodiments;
FIG. 1C illustrates cross section of a second sensing region of the multi-core optical fiber instrument of FIG. 1A, in accordance with some embodiments;
FIG. 2A is an exemplary embodiment of a structure of the first sensing region the multi-core optical fiber instrument of FIG. 1A, in accordance with some embodiments;
FIG. 2B is an exemplary embodiment of a structure of the second sensing region the multi-core optical fiber instrument of FIG. 1A, in accordance with some embodiments;
FIG. 2C is an exemplary embodiment of a structure of the multi-core optical fiber instrument of FIG. 1A illustrating a set of third optical fiber cores, in accordance with some embodiments; and
FIG. 3 is an illustrative embodiment of a medical device monitoring system including a multi-core optical fiber instrument with optic sensing capabilities, in accordance with some embodiments.

### DETAILED DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

The directional terms "proximal" and "distal" are used herein to refer to opposite locations on a medical device. The proximal end of the device is defined as the end of the device closest to the end-user when the device is in use by the end-user. The distal end is the end opposite the proximal end, along the longitudinal direction of the device, or the end furthest from the end-user.

The term "logic" may be representative of hardware, firmware or software that is configured to perform one or more functions. As hardware, the term logic may refer to or include circuitry having data processing and/or storage functionality. Examples of such circuitry may include, but are not limited or restricted to a hardware processor (e.g., microprocessor, one or more processor cores, a digital signal processor, a programmable gate array, a microcontroller, an application specific integrated circuit "ASIC", etc.), a semiconductor memory, or combinatorial elements.

Additionally, or in the alternative, the term logic may refer to or include software such as one or more processes, one or more instances, Application Programming Interface(s) (API), subroutine(s), function(s), applet(s), servlet(s), routine(s), source code, object code, shared library/dynamic link library (dll), or even one or more instructions. This software may be stored in any type of a suitable non-transitory storage medium, or transitory storage medium (e.g., electrical, optical, acoustical or other form of propagated signals such as carrier waves, infrared signals, or digital signals). Examples of a non-transitory storage medium may include, but are not limited or restricted to a programmable circuit; non-persistent storage such as volatile memory (e.g., any type of random access memory "RAM"); or persistent storage such as non-volatile memory (e.g., read-only memory "ROM", power-backed RAM, flash memory, phase-change memory, etc.), a solid-state drive, hard disk drive, an optical disc drive, or a portable memory device. As firmware, the logic may be stored in persistent storage.

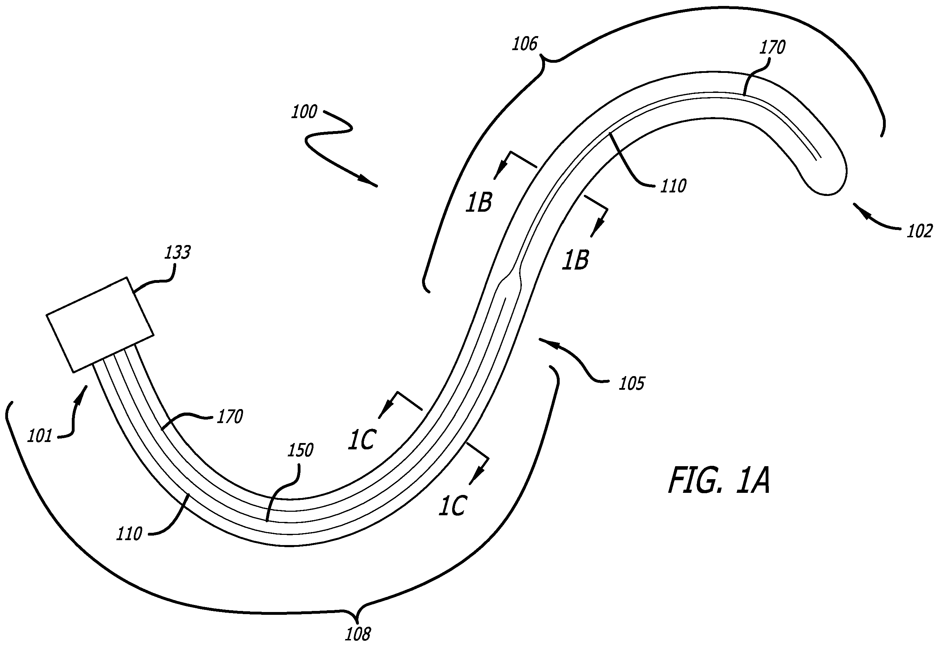

FIG. 1A is an illustrative embodiment (not according to the invention) of an elongate multi-core optical fiber instrument (instrument) 100 configured for insertion into a patient body, the instrument 100 defining a proximal end 101 and a distal end 102. The instrument 100 may be incorporated into a medical device. In some embodiments, the medical device may be vascular device such as a guidewire, a stylet, a catheter, or any other device configured for insertion into a vasculature of a patient. In other embodiments, the medical device may be a probe, a needle, a scope, biopsy device, or other device for insertion into a patient generally.

The instrument 100 may include multiple sets of optical fiber cores. The optical fiber cores are coupled to a fiber optic connector 133 at the proximal end 101 and extend along the instrument 100. The fiber cores may extend along an entire length of the instrument or a portion thereof. The various sets of optical fiber cores may define different sensing capabilities of the instrument 100. For example, one or more sets of optical fiber cores may be configured to determine one or more physical parameters of the instrument 100. The physical parameters may include a shape, a tensile strain, a compressive strain, a torsional strain, a curve, a bend radius, and a motion, for example. In some instances, sensing the shape of the instrument 100 may facilitate detection of an azygos blood vessel. Similarly, one or more sets of optical fiber cores may be configured to determine one or more physiological parameters of the patient body. Exemplary physiological parameters may include a temperature, a fluid flow rate or velocity, a motion of body tissue, and a pulse oximetry.

According to one embodiment, the instrument 100 includes a first sensing region 106 and a second sensing region 108 including a transition point 105 therebetween. The first sensing region 106 may extend between the distal end 102 and the transition point 105 and the second sensing region 108 may extend between the transition point 105 and the proximal end 101. In other embodiments, the instrument 100 may be divided into three or more sensing regions. In some embodiments, the first and second regions 106, 108 may partially overlap across the transition point 105. In the illustrated embodiment, each of the sensing regions 106, 108 is configured for determining a shape of the instrument 100 along the respective sensing region.

The shape sensing capability of the instrument 100 is provided via a set of first optical fiber cores 110 and a set of second optical fiber cores 150. In the illustrated embodiment, the first fiber cores 110 extend from the proximal end 101 to the distal end 102, and the second fiber cores 150 extend from the proximal end 101 to the transition point 105. As such, the first fiber cores 110 extend from the connector 133 through both the second sensing region 108 and the first sensing region 106. In some embodiments, the instrument 100 may include additional sets of optical fiber cores.

According to the illustrated embodiment, a set of third optical fiber cores 170 provides for a physiological parameter sensing capability along the instrument 100. The third fiber cores 170 extend along the length instrument 100.

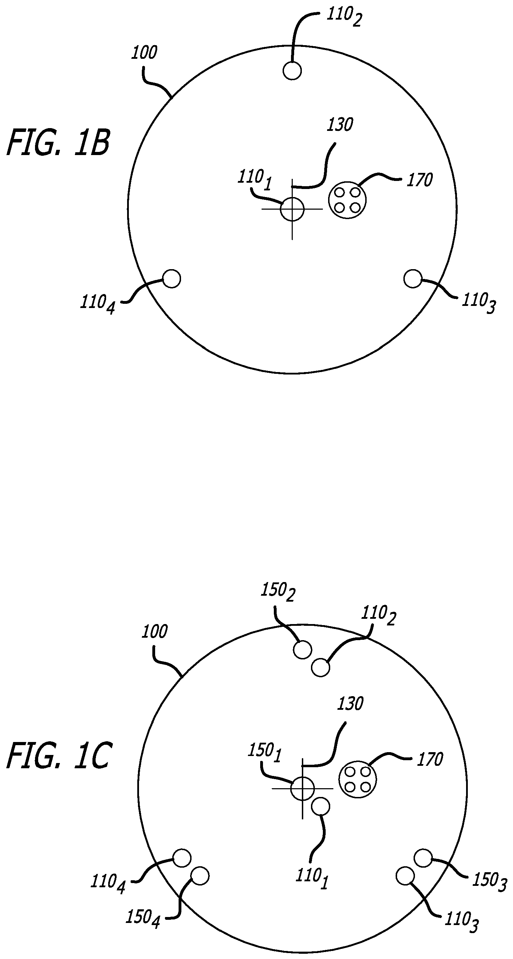

FIG. 1B illustrates a cross-section of the instrument 100 (not according to the invention) cut along sectioning lines 1B-1B across the first sensing region 106. As shown, the instrument 100 may define a circular cross section. As shown, the first fiber cores 110 include four optical fiber cores, i.e., fiber cores 110₁-101₄. In other embodiments, the first fiber cores 110 may include less than four optical fiber cores or more than four optical fiber cores. The fiber core 110₁ is disposed at a central axis 130 of the instrument 100 and the fiber cores 110₂-110₄ are disposed adjacent a circumferential surface of the instrument 100. The fiber cores 110₂-110₄ may be positioned substantially equidistant from each other as measured along a circumference of the instrument 100, such as at "top" (12 o'clock), "bottom-left" (8 o'clock) and "bottom-right" (4 o'clock) locations as shown. The third optical fiber cores 170 are also shown disposed within the cross section of the first sensing region 106.

FIG. 1C illustrates a cross section of the instrument 100 cut along sectioning lines 1C-1C across the second sensing region 108. As shown, the fiber core 150₁ is disposed at the central axis 130 of the instrument 100 and the fiber cores 150₂-150₄ are disposed adjacent the circumferential surface of the instrument 100. The fiber cores 150₂-150₄ may be positioned substantially equidistant from each other as measured along the circumference of the instrument 100, such as at "top" (12 o'clock), "bottom-left" (8 o'clock) and "bottom-right" (4 o'clock) locations as shown. Also shown are the fiber cores 110₁-110₄ passing through the second sensing region 108 immediately adjacent the fiber cores 150₁-150₄. As such, the fiber cores 110₁ and 150₁ may both be positioned substantially at the central axis 130 along the second sensing region 108. Similarly, the fiber cores 110₂ -110₄ and the fiber cores 150₂-150₄ may also be positioned substantially at the "top" (12 o'clock), the "bottom-left" (8 o'clock) and the "bottom-right" (4 o'clock) locations, respectively along the second sensing region 108. The third optical fiber cores 170 are also shown disposed within the cross section of the second sensing region 108.

In an embodiment where the instrument 100 is incorporated within a tubular medical device, the fiber cores 110₂-110₄ and the fiber cores 150₂-150₄ may be disposed within a tubular wall. In such embodiments, the tubular medical device may include a rib extending through the central axis of the tubular device in which the fiber cores 110₁, 150₁ may be disposed.

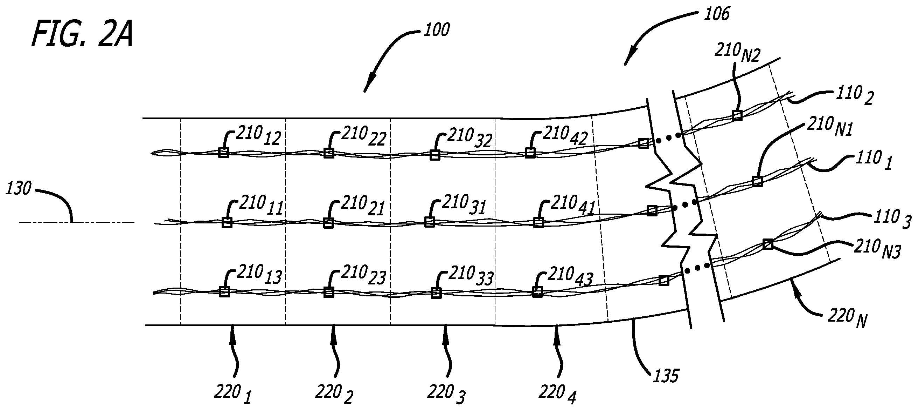

FIG. 2A is an exemplary embodiment of a structure of the first sensing region 106. The first sensing region 106 includes fiber cores 110₁-110₄ (fiber core 110₄ not shown) extending longitudinally along the first sensing region 106. As shown, the first sensing region 106 is subdivided into a plurality of cross-sectional regions 220₁-220_{N}. Some or all of the cross-sectional regions 220₁...220_{N} may be static (e.g., equal in length) or may be dynamic (e.g., vary in length among the regions 220₁...220_{N}). Each of the of cross-sectional regions 220₁-220_{N} includes a sensor 210 disposed within each of the fiber cores 110₁-110₄ extending therethrough. In FIG. 2A, each of the sensors 210 includes a cross-sectional region designation "m" (m=1...M) and a fiber designation "n" (n=1...N) shown as 210ₘₙ. For example, sensor 210₂₃ is located in the cross-sectional region 220₂ and within fiber core 110₃. The third optical fiber cores 170 extend along the first sensing region 106. However, the third optical fiber cores 170 are not shown in FIG. 2A for purposes of clarity.

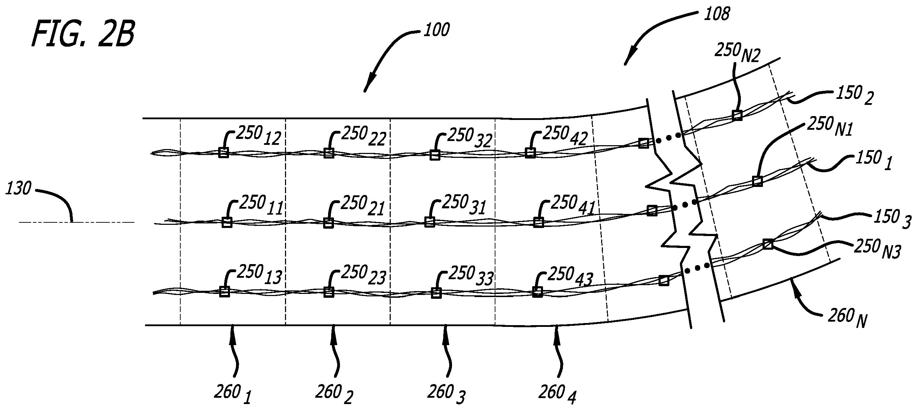

FIG. 2B is an exemplary embodiment of a structure of the second sensing region 108. The second sensing region 108 includes fiber cores 150₁-150₄ (150₄ not shown) extending longitudinally along the second sensing region 108. First fiber cores 110₁-110₄ (not shown for clarity purposes) also pass through the second sensing region 108. Similar to the first sensing region 106 as described above in relation to FIG. 2A, the second sensing region 108 is divided into a plurality of cross-sectional regions 260₁-260_{N}. Some or all of the cross-sectional regions 260₁...260_{N} may be static or dynamic as defined above. Each of the of cross-sectional regions 260₁-260_{N} includes a sensor 250 disposed within each of the fiber cores 150₁-150₄ extending therethrough. The sensors 250 include a cross-sectional region designation "m" and a fiber designation "n" shown as 210ₘₙ. The fiber cores 110₁-110₄ may simply pass through the second sensing region 108 and therefore, the cross-sectional regions 260₁-260_{N} do not include sensors 210 disposed within the fiber cores 110₁-110₄. The third optical fiber cores 170 also extend along the second sensing region 108. However, the third optical fiber cores 170 are not shown in FIG. 2B for clarity purposes.

In some embodiments, the second sensing region 108 may form a proximal extension of the first sensing region 106, e.g., the first sensing region 106 and the second sensing region 108 may define the same or similar shape sensing characteristics. For example, as described above, the circumferential spacing of the second fiber cores 150₁-150₄ along the second sensing region 108 may be consistent with the circumferential spacing of the first fiber cores 110₁-110₄ along the first sensing region 106. In similar fashion, the cross-sectional regions 260₁-260_{N} may be consistent with the cross-sectional regions 220₁-220_{N}.

In some embodiments, the cross-sectional regions 260₁-260_{N} may be different than the cross-sectional regions 220₁-220_{N}. For example, the cross-sectional regions 220₁-220_{N} may be shorter than the cross-sectional regions 260₁-260_{N}. A shorter cross-sectional region may define an enhanced parameter sensing resolution along the first sensing region 106. In some instances, an increased shape sensing resolution along the first sensing region 106 may be advantageous in embodiments where the instrument 100 is a vascular device configured to traverse blood vessels near the heart of the patient.

The instrument 100 is configured to operate across a spectrum of light (i.e., a range of light wavelength). More specifically, each optical fiber is configured to operate across the spectrum. The spectrum may be divided up into spectral widths (i.e., portions of the spectrum), where each spectral width defines a center wavelength. As discussed above, the sensors 210 and 250 may be reflective gratings such as fiber Bragg gratings (FBG), where each sensor along a single fiber is configured to reflect light within a defined different spectral width. For example, sensors 210₁₁-210_{N1} disposed along fiber core 110₁ are configured to reflect light within different spectral widths of the spectrum. In some embodiments, the spectral widths defined for one optical fiber may be the same as the spectral widths defined for another optical fiber. For example, the sensors 210₁₂-210_{N2} disposed along fiber core 110₂ may be configured to reflect light within the same spectral widths as sensors 210₁₁-210_{N1}, respectively. In some embodiments, the defined spectral widths may be the same fiber cores as first fiber cores 110₁-110₄, second fiber cores 150₁-150₄, and third fiber cores 170.

As stated above, the sensors disposed within the different fiber cores 110₂-110₃ but at the same cross-sectional regions 220₁-220_{N} may be configured to reflect incoming light at the same (or a substantially similar) center wavelength. As a result, the reflected light returns information that allows for a determination of one or more parameters according to a wavelength shift away from the center wavelength. For example, a strain (e.g., compression or tension) applied to an optical fiber (e.g., the fiber core 110₁ along the first sensing region 106) results in a wavelength shift associated with the returned, reflected light. More specifically, a strain of the portion of the fiber core 110₁ at the location of the sensor 210₁₁ causes the wavelength of light reflected by the sensor 210₁₁ to be shifted away from the center wavelength for the sensor 210₁₁. By way of general summary, a given sensor may reflect light at a wavelength shifted away from the defined center wavelength in accordance with a parameter experienced by the sensor.

According to one embodiment and by way of example, with respect to the first sensing region 106, in response to a curve of the instrument 100 toward the left direction (see FIG. 1B), the fiber core 110₄ with the shortest radius in curved state would experience compressive strain (shortening). At the same time, the fiber core 110₃ with the longest radius in the curved state would experience tensile strain (lengthening). As these strains are different, the reflected light from sensors 210ₙ₃ and 210ₙ₄ associated with the fiber cores 110₃ and 110₄ will reflect light having different shifts in wavelength. The differences in wavelength shift of the reflected light signals 152 can be used to determine the curve or shape of the instrument 100 corresponding to the wavelength shifts caused by the compressive strain and tensile strain for fiber cores 110₄,110₃, respectively in comparison to the wavelength shift of the centrally located fiber core 110₁.

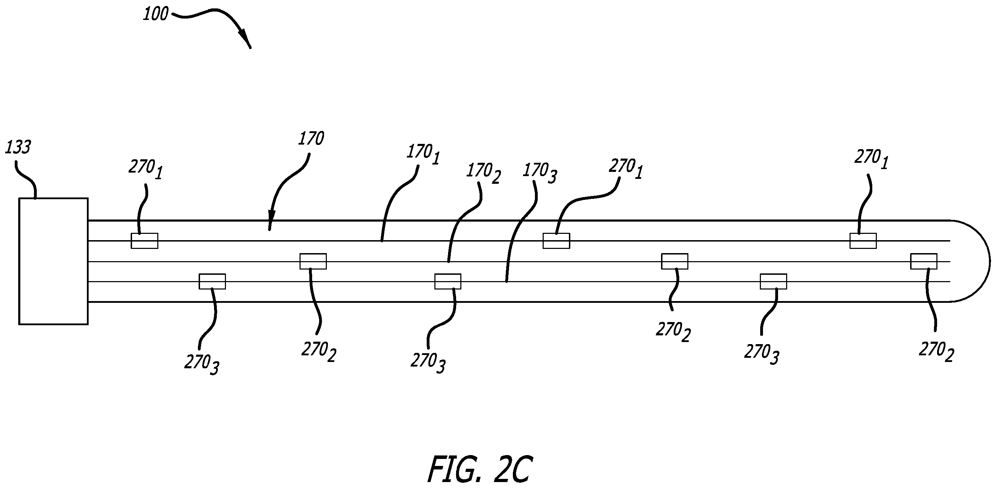

FIG. 2C illustrates the set of third fiber cores 170 extending along the instrument 100 (not according to the invention) from the proximal end 101 to the distal end 102. As discussed above, the third fiber cores 170 may be configured to determine one or more physiological parameters of the patient body. In the illustrated exemplary embodiment, the set of third fiber cores 170 includes three fiber cores 170₁, 170₂, and 170₃. In other embodiments, the set of third fiber cores 170 may include more or less than three fiber coress. The third fiber cores 170 extend through the first and second sensing regions 106, 108 as described above so as to determine physiological parameters along the first and second sensing regions 106, 108. In some embodiments, each third fiber 170 may be configured to determine a single physiological parameter. In other embodiments, each third fiber 170 may be configured to determine more than one physiological parameter. In still other embodiments, a physiological parameter may be determined by a combination of third fiber cores 170.

According to one embodiment, each of the third fiber cores 170 may determine a single physiological parameter as described below. The fiber 170₁ may be configured to determine a temperature at various locations along the instrument 100. As such the fiber 170₁ includes one or more sensors 270₁ disposed at defined locations along the fiber 170₁ where the sensors 270₁ are configured to measure a temperature. The fiber 170₂ may be configured to determine a blood flow rate at various locations along the instrument 100. As such the fiber 170₂ includes one or more sensors 270₂ disposed at defined locations along the fiber 170₂ where the sensors 270₂ are configured to measure a blood flow rate via a doppler effect, for example. The fiber 170₃ may be configured to determine a pulse oximetry at various locations along the instrument 100. As such the fiber 170₃ includes one or more sensors 270ₛ disposed at defined locations along the fiber 170₃, where the sensors 270₃ are configured to measure the pulse oximetry.

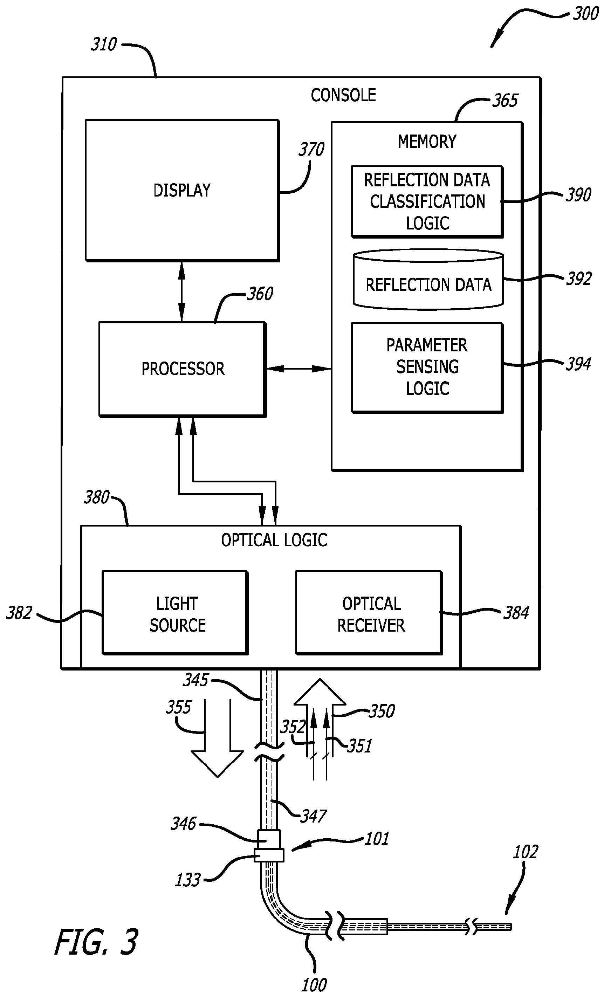

FIG. 3 is an illustrative embodiment of a medical system 300 including the instrument 100 for sensing parameters experienced by an elongate medical device inserted into a patient body. As shown, the system 300 generally includes a console 310 and the medical instrument 100 communicatively coupled with the console 310. The instrument 100 is incorporated into or coupled with an elongate medical device where the elongate medical device is one of an introducer wire, a guidewire, a stylet, a probe, a sheath, a mesh, a tube, an obturator, or a catheter.

The optical connector 133 enables the instrument 100 to be operably connected to the console 310 via an interconnect 345 including one or more interconnecting optical fiber cores 347. The connector 346 is configured to operatively couple with the optical connector 133 to allow for the propagation of light between the console 310 and the instrument 100.

An exemplary implementation of the console 310 includes a processor 360, a memory 365, a display 370 and optical logic 380, although it is appreciated that the console 310 can take any one of a variety of forms and may include additional components (e.g., power supplies, ports, interfaces, etc.) that may facilitate operation of the console 310. The processor 360, with access to the memory 365 (e.g., non-volatile memory or non-transitory, computer-readable medium), is included to control functionality of the console 310 during operation. As shown, the display 370 may be a liquid crystal diode (LCD) display integrated into the console 310 and employed as a user interface to display information to the clinician during use. In another embodiment, the display 370 may be separate from the console 310. Although not shown, a user interface may be configured to provide user control of the console 310.

Referring still to FIG. 3, the optical logic 380 is configured to support operability of the instrument 100 including the return of information to the console 310, which information may facilitate determining the one or more parameters experienced by the instrument 100. Characteristics of reflected light signals 350 received at the console 310 from the instrument 100 correlate with the parameters experienced by the instrument 100. The characteristics may include shifts in wavelength caused by the parameters experienced by the instrument 100, as further described below. From information associated with the reflected light signals 350, the console 110 may determine (through computation or extrapolation of the wavelength shifts) the parameters experienced by the instrument 100.

According to one embodiment of the disclosure, as shown in FIG. 3, the optical logic 380 may include a light source 382 and an optical receiver 384. The light source 382 is configured to transmit the incident light 355 (e.g., broadband) for propagation over the interconnecting optical fiber cores 347 included in the interconnect 345, which are optically connected to the optical fiber cores 110, 150, 170 of the instrument 100. In one embodiment, the light source 382 is a tunable swept laser, although other suitable light sources can also be employed in addition to a laser, including semi-coherent light sources, LED light sources, etc.

The optical receiver 384 is configured to: (i) receive reflected optical signals 352 (reflected light signals) received from sensors fabricated within each of the fiber cores 110, 150, 170 of the instrument 100, and (ii) translate the reflected light signals 352 into reflection data 392, namely data in the form of electrical signals representative of the reflected light signals 352 including wavelength shifts caused by parameters determined by the instrument 100. Herein, the optical receiver 384 may be implemented as a multi-channel photodetector, such as a positive-intrinsic-negative "PIN" photodiode, avalanche photodiode, or the like.

As shown, both the light source 382 and the optical receiver 384 are operably connected to the processor 360, which governs their operation. Also, the optical receiver 384 is operably coupled to provide the reflection data 392 to the memory 365 for storage and processing by reflection data classification logic 390. The reflection data classification logic 390 may be configured to identify which reflection data 392 pertains to the first fiber cores 120, the second fiber cores 150, and the third fiber cores 170. The reflection data classification logic 390 may also identify the reflection data 392 pertaining to the sensors corresponding to the cross-sectional regions along the respective fiber cores. The reflection data 392 for each sensor is made available to parameter sensing logic 394 for analytics.

According to one embodiment of the disclosure, the parameter sensing logic 394 is configured to determine a shape of the instrument 100 along the first sensing region 106 via analysis of the reflected data 392 associated with the first optical fiber cores 120. Similarly, the parameter sensing logic 394 is configured to determine a shape of the instrument 100 along the second sensing region 108 via analysis of the reflected data 392 associated with the second optical fiber cores 150. From these analytics, the parameter sensing logic 194 may determine the shape of the instrument 100 (or more specifically the shape of the first and second sensing regions 106, 108) in 3D space for rendering on the display 370.

The parameter sensing logic 394 may also be configured to analyze reflection data 392 received from one or more sensors 270 disposed along the third optical fiber cores 170 to determine the physiological parameters of the patient body at the sensor locations along the instrument 100 for rendering on the display 370.

While some particular embodiments have been disclosed herein, and while the particular embodiments have been disclosed in some detail, it is not the intention for the particular embodiments to limit the scope of the concepts provided herein. Additional adaptations and/or modifications can appear to those of ordinary skill in the art, and, in broader aspects, these adaptations and/or modifications are encompassed as well. Accordingly, departures may be made from the particular embodiments disclosed herein without departing from the scope of the concepts provided herein. The scope of the invention is defined by the claims.

## Claims

1. An elongate multi-core optical fiber instrument (100) for insertion within a patient body, the instrument (100) comprising:
a set of first optical fiber cores (110) extending along a first sensing region (106) of the multi-core optical fiber instrument (100), each first optical fiber core (110) comprising a set of first reflective gratings (210) disposed along the first sensing region (106);
a set of second optical fiber cores (150) extending along a second sensing region (108) of the multi-core optical fiber instrument (100), each second optical fiber core (150) comprising a set of second reflective gratings (250) disposed along the second sensing region (108), wherein:
the first sensing region (106) is located distal the second sensing region (108),
the first optical fiber cores (110) extend along the second sensing region (108),
wherein the multi-core optical fiber instrument (100) defines a circular cross section along the first region,
wherein at least three of the first optical fiber cores (110) are disposed adjacent a circumferential surface of the multi-core optical fiber instrument (100) along the first region,
wherein the multi-core optical fiber instrument (100) defines a circular cross section along the second sensing region (108), and wherein at least three of the second optical fiber cores (150) are disposed adjacent a circumferential surface of the multi-core optical fiber instrument (100) along the second region, and **characterised in that**
at least one of the at least three second optical fiber cores (150) is disposed at the same angular position as one of the at least three first optical fiber cores (110).

2. The multi-core optical fiber instrument (100) of claim 1, wherein:
the set of first reflective gratings (210) is configured to operate across a set of first spectral widths of a light spectrum such that each first reflective gratings (210) of one first fiber core operates across a different spectral width from the other first reflective gratings (210) of the one first fiber core, and
the set of second reflective gratings (250) is configured to operate across a set of second spectral widths of the light spectrum such that each second reflective gratings (250) of one second fiber core operates across a different spectral width from the other second reflective gratings (250) of the one second fiber core,
preferably wherein the set of first spectral widths are the same for each first fiber core, and the set of second spectral widths are the same for each second fiber core.

3. The multi-core optical fiber instrument (100) of claim 2, wherein a portion of the second spectral widths are a subset of the first spectral widths.

4. The multi-core optical fiber instrument (100) of either claim 2 or claim 3, wherein a majority of the set of second spectral widths are a subset of the first spectral widths.

5. The multi-core optical fiber instrument (100) of any of claims 2-4, wherein a substantial entirety of the second spectral widths are a subset of the first spectral widths.

6. The multi-core optical fiber instrument (100) of any of the preceding claims, wherein:
the first reflective gratings (210) are configured to determine one or more physical parameters of the multi-core optical fiber instrument (100) along the first sensing region (106), and
the second reflective gratings (250) are configured to determine the one or more physical parameter of the multi-core optical fiber instrument (100) along the second sensing region (108),
preferably wherein the physical parameters comprise at least one of a bending direction, a shape, torsional strain, longitudinal strain, and a motion of the multi-core optical fiber instrument (100).

7. The multi-core optical fiber instrument (100) of any of the preceding claims,
wherein the at least three first optical fiber cores (110) are equally spaced along the circumference surface,
preferably wherein the at least three first optical fiber cores (110) extend along the first region at a constant angular position with respect to the instrument.

8. The multi-core optical fiber instrument (100) of any of the preceding claims, wherein the at least three second optical fiber cores (150) are equally spaced along the circumference surface of the second region,
preferably wherein the at least three second optical fiber cores (150) extend along the second sensing region (108) at a constant angular position with respect to the multi-core optical fiber instrument (100).

9. The multi-core optical fiber instrument (100) of claim 8, wherein each of the at least three second optical fiber cores (150) is disposed at the same angular position as a separate one of the at least three first optical fiber cores (110).

10. The multi-core optical fiber instrument (100) of any of the preceding claims, wherein the second sensing region (108) at least partially overlaps the first sensing region (106),
preferably wherein at least one of the first optical fiber cores (110) is disposed adjacent the circumferential surface along the second sensing region (108).

11. The multi-core optical fiber instrument (100) of any of the preceding claims,
wherein the multi-core optical fiber instrument (100) is incorporated into one of an introducer wire, a guidewire, a stylet, a probe, a sheath, a mesh, a tube, an obturator, or a catheter.

12. A medical system for sensing one or more parameters experienced by an elongate medical device inserted within a patient body, the system comprising:
the elongate multi-core optical fiber instrument (100) according to any of the preceding claims, wherein:
the multi-core optical fiber instrument (100) is incorporated into the elongate medical device, and
the medical device is one of an introducer wire, a guidewire, a stylet, a probe, a sheath, a mesh, a tube, an obturator, or a catheter; and
a console (310) including optical logic (380) including a light source (382) and an optical receiver (384), one or more processors (360) and a non-transitory computer-readable medium having stored thereon logic that, when executed by the one or more processors, causes operations including:
providing an incident light signal to the multi-core optical fiber instrument (100),
receiving reflected light signals from the first reflective gratings (210) across a set of first spectral widths,
receiving reflected light signals from the second reflective gratings (250) across a set of second spectral widths, the second spectral widths being a subset of the first spectral widths,
processing the reflected light signals associated with the first reflective gratings (210) and the second reflective gratings (250),
determining a shape of the multi-core optical fiber instrument (100) along the first sensing region (106) from reflected light associated with the first reflective gratings (210),
determining a shape of the multi-core optical fiber instrument (100) along the second sensing region (108) from reflected light associated with the second reflective gratings (250), and
causing a rendering on a graphical display of the shape of the multi-core optical fiber instrument (100) along the first sensing region (106) and the second sensing region (108).

13. The system of claim 12, wherein the multi-core optical fiber instrument (100) further comprises a set of third optical fiber cores (170) extending along the multi-core optical fiber instrument (100), each third optical fiber core (170) comprising at least one third sensor, and wherein the logic, when executed by the one or more processors (360), further causes operations including:
receiving reflected light signals from the at least one third sensor across a first spectral width,
determining a physiological parameter of the patient body from reflected light associated with the at least one third sensor, and
causing a rendering on the graphical display depicting the physiological parameter along the multi-core optical fiber instrument (100) in combination with the shape of the multi-core optical fiber instrument (100) along the first sensing region (106) and the second sensing region (108).

14. The system of claim 13, wherein the physiological parameter is one of a body temperature, a blood flow rate, or a pulse oximetry.

## Patentansprüche

1. Längliches optisches Mehrkernfaserinstrument (100) zum Einführen in einen Körper eines Patienten, wobei das Instrument (100) Folgendes umfasst:
einen Satz von ersten optischen Faserkernen (110), die sich entlang eines ersten Erfassungsbereichs (106) des optischen Mehrkernfaserinstruments (100) erstrecken, wobei jeder erste optische Faserkern (110) einen Satz von ersten reflektierenden Gittern (210) umfasst, die entlang des ersten Erfassungsbereichs (106) angeordnet sind;
einen Satz von zweiten optischen Faserkernen (150), die sich entlang eines zweiten Erfassungsbereichs (108) des optischen Mehrkernfaserinstruments (100) erstrecken, wobei jeder zweite optische Faserkern (150) einen Satz von zweiten reflektierenden Gittern (250) umfasst, die entlang des zweiten Erfassungsbereichs (108) angeordnet sind, wobei:
der erste Erfassungsbereich (106) sich distal zu dem zweiten Erfassungsbereich (108) befindet,
die ersten optischen Faserkerne (110) sich entlang des zweiten Erfassungsbereichs (108) erstrecken,
wobei das optische Mehrkernfaserinstrument (100) entlang des ersten Bereichs einen kreisförmigen Querschnitt definiert,
wobei mindestens drei der ersten optischen Faserkerne (110) entlang des ersten Bereichs angrenzend an eine Umfangsfläche des optischen Mehrkernfaserinstruments (100) angeordnet sind,
wobei das optische Mehrkernfaserinstrument (100) entlang des zweiten Erfassungsbereichs (108) einen kreisförmigen Querschnitt definiert und wobei mindestens drei der zweiten optischen Faserkerne (150) entlang des zweiten Bereichs angrenzend an eine Umfangsfläche des optischen Mehrkernfaserinstruments (100) angeordnet sind, und **dadurch gekennzeichnet, dass** mindestens einer der mindestens drei zweiten optischen Faserkerne (150) in derselben Winkelposition wie einer der mindestens drei ersten optischen Faserkerne (110) angeordnet ist.

2. Optisches Mehrkernfaserinstrument (100) nach Anspruch 1, wobei:
der Satz von ersten reflektierenden Gittern (210) dazu ausgebildet ist, über einen Satz von ersten Spektralbreiten eines Lichtspektrums hinweg zu arbeiten, sodass jedes erste reflektierende Gitter (210) eines ersten Faserkerns über eine andere Spektralbreite hinweg arbeitet als die anderen ersten reflektierenden Gitter (210) des einen ersten Faserkerns, und
der Satz von zweiten reflektierenden Gittern (250) dazu ausgebildet ist, über einen Satz von zweiten Spektralbreiten des Lichtspektrums zu arbeiten, sodass jedes zweite reflektierende Gitter (250) eines zweiten Faserkerns über eine andere Spektralbreite hinweg arbeitet als die anderen zweiten reflektierenden Gitter (250) des einen zweiten Faserkerns,
wobei vorzugsweise der Satz von ersten Spektralbreiten für jeden ersten Faserkern gleich ist und der Satz von zweiten Spektralbreiten für jeden zweiten Faserkern gleich ist.

3. Optisches Mehrkernfaserinstrument (100) nach Anspruch 2, wobei ein Abschnitt der zweiten Spektralbreiten ein Teilsatz der ersten Spektralbreiten ist.

4. Optisches Mehrkernfaserinstrument (100) nach Anspruch 2 oder Anspruch 3, wobei ein Mehrheit des Satzes von zweiten Spektralbreiten ein Teilsatz der ersten Spektralbreiten ist.

5. Optisches Mehrkernfaserinstrument (100) nach einem der Ansprüche 2-4, wobei im Wesentlichen sämtliche der zweiten Spektralbreiten ein Teilsatz der ersten Spektralbreiten ist.

6. Optisches Mehrkernfaserinstrument (100) nach einem der vorstehenden Ansprüche, wobei:
die ersten reflektierenden Gitter (210) dazu ausgebildet sind, einen oder mehrere physikalische Parameter des optischen Mehrkernfaserinstruments (100) entlang des ersten Erfassungsbereichs (106) zu bestimmen, und
die zweiten reflektierenden Gitter (250) dazu ausgebildet sind, den einen oder mehrere physikalische Parameter des optischen Mehrkernfaserinstruments (100) entlang des zweiten Erfassungsbereichs (108) zu bestimmen,
wobei die physikalischen Parameter vorzugsweise mindestens eines von einer Biegerichtung, einer Form, einer Torsionsdehnung, einer Längsdehnung und einer Bewegung des optischen Mehrkernfaserinstruments (100) umfassen.

7. Optisches Mehrkernfaserinstrument (100) nach einem der vorstehenden Ansprüche,
wobei die mindestens drei ersten optischen Faserkerne (110) entlang der Umfangsfläche gleich beabstandet angeordnet sind,
wobei vorzugsweise die mindestens drei ersten optischen Faserkerne (110) sich entlang des ersten Bereichs in einer konstanten Winkelposition in Bezug auf das Instrument erstrecken.

8. Optisches Mehrkernfaserinstrument (100) nach einem der vorstehenden Ansprüche, wobei die mindestens drei zweiten optischen Faserkerne (150) entlang der Umfangsfläche des zweiten Bereichs gleich beabstandet sind,
wobei vorzugsweise die mindestens drei zweiten optischen Faserkerne (150) sich entlang des zweiten Erfassungsbereichs (108) in einer konstanten Winkelposition in Bezug auf das optische Mehrkernfaserinstrument (100) erstrecken.

9. Optisches Mehrkernfaserinstrument (100) nach Anspruch 8, wobei jeder der mindestens drei zweiten optischen Faserkerne (150) an derselben Winkelposition wie ein separater der mindestens drei ersten optischen Faserkerne (110) angeordnet ist.

10. Optisches Mehrkernfaserinstrument (100) nach einem der vorstehenden Ansprüche, wobei der zweite Erfassungsbereich (108) mindestens teilweise den ersten Erfassungsbereich (106) überlappt,
wobei vorzugsweise mindestens einer der ersten optischen Faserkerne (110) entlang des zweiten Erfassungsbereichs (108) angrenzend an die Umfangsfläche angeordnet ist.

11. Optisches Mehrkernfaserinstrument (100) nach einem der vorstehenden Ansprüche, wobei das optische Mehrkernfaserinstrument (100) in eines von einem Einführdraht, einem Führungsdraht, einem Mandrin, einer Sonde, einer Hülle, einem Netz, einem Schlauch, einem Obturator oder einem Katheter integriert ist.

12. Medizinisches System zum Erfassen eines oder mehrerer Parameter, die durch eine längliche medizinische Vorrichtung erfahren werden, die in einen Patientenkörper eingeführt ist, wobei das System Folgendes umfasst:
das längliche optische Mehrkernfaserinstrument (100) nach einem der vorstehenden Ansprüche, wobei:
das optische Mehrkernfaserinstrument (100) in die längliche medizinische Vorrichtung integriert ist, und
die medizinische Vorrichtung eines von einem Einführdraht, einem Führungsdraht, einem Mandrin, einer Sonde, einer Hülle, einem Netz, einem Schlauch, einem Obturator oder einem Katheter ist; und
eine Konsole (310), die eine optische Logik (380), die eine Lichtquelle (382) und einen optischen Empfänger (384) einschließt, einen oder mehrere Prozessoren (360) und ein nichttransitorisches computerlesbares Medium einschließt, auf dem eine Logik gespeichert ist, die bei Ausführung durch den einen oder die mehreren Prozessoren Operationen veranlasst, einschließlich:
Bereitstellen eines einfallenden Lichtsignals an das optische Mehrkernfaserinstrument (100),
Empfangen von reflektierten Lichtsignalen von den ersten reflektierenden Gittern (210) über einen Satz von ersten Spektralbreiten hinweg,
Empfangen von reflektierten Lichtsignalen von den zweiten reflektierenden Gittern (250) über einen Satz von zweiten Spektralbreiten hinweg, wobei die zweiten Spektralbreiten ein Teilsatz der ersten Spektralbreiten sind,
Verarbeiten der reflektierten Lichtsignale, die den ersten reflektierenden Gittern (210) und den zweiten reflektierenden Gittern (250) zugeordnet sind,
Bestimmen einer Form des optischen Mehrkernfaserinstruments (100) entlang des ersten Erfassungsbereichs (106) aus reflektiertem Licht, das den ersten reflektierenden Gittern (210) zugeordnet ist,
Bestimmen einer Form des optischen Mehrkernfaserinstruments (100) entlang des zweiten Erfassungsbereichs (108) aus reflektiertem Licht, das den zweiten reflektierenden Gittern (250) zugeordnet ist, und
Veranlassen einer Wiedergabe der Form des optischen Mehrkernfaserinstruments (100) entlang des ersten Erfassungsbereichs (106) und des zweiten Erfassungsbereichs (108) auf einer graphischen Anzeige.

13. System nach Anspruch 12, wobei das optische Mehrkernfaserinstrument (100) weiter einen Satz von dritten optischen Faserkernen (170) umfasst, die sich entlang des optischen Mehrkernfaserinstruments (100) erstrecken, wobei jeder dritte optische Faserkern (170) mindestens einen dritten Sensor umfasst und wobei die Logik bei Ausführung durch den einen oder mehrere Prozessoren (360) weiter Operationen veranlasst, einschließlich:
Empfangen von reflektierten Lichtsignalen von dem mindestens einen dritten Sensor über eine erste Spektralbreite hinweg,
Bestimmen eines physiologischen Parameters des Patientenkörpers aus reflektiertem Licht, das dem mindestens einen dritten Sensor zugeordnet ist, und
Veranlassen einer Wiedergabe, die den physiologischen Parameter entlang des optischen Mehrkernfaserinstruments (100) in Kombination mit der Form des optischen Mehrkernfaserinstruments (100) entlang des ersten Erfassungsbereichs (106) und des zweiten Erfassungsbereichs (108) abbildet, auf der graphischen Anzeige.

14. System nach Anspruch 13, wobei der physiologische Parameter einer von einer Körpertemperatur, einer Blutflussrate oder einer Pulsoximetrie ist.

## Revendications

1. Instrument (100) à fibre optique à âmes multiples allongé pour insertion dans un corps de patient, l'instrument (100) comprenant :
un ensemble de premières âmes (110) de fibre optique s'étendant le long d'une première région de détection (106) de l'instrument (100) à fibre optique à âmes multiples, chaque première âme (110) de fibre optique comprenant un ensemble de premiers réseaux réfléchissants (210) disposés le long de la première région de détection (106) ;
un ensemble de deuxièmes âmes (150) de fibre optique s'étendant le long d'une deuxième région de détection (108) de l'instrument (100) à fibre optique à âmes multiples, chaque deuxième âme (150) de fibre optique comprenant un ensemble de deuxièmes réseaux réfléchissants (250) disposés le long de la deuxième région de détection (108), dans lequel :
la première région de détection (106) est située de manière distale par rapport à la deuxième région de détection (108),
les premières âmes (110) de fibre optique s'étendent le long de la deuxième région de détection (108),
dans lequel l'instrument (100) à fibre optique à âmes multiples définit une section transversale circulaire le long de la première région,
dans lequel au moins trois des premières âmes (110) de fibre optique sont disposées adjacentes à une surface circonférentielle de l'instrument (100) à fibre optique à âmes multiples le long de la première région,
dans lequel l'instrument (100) à fibre optique à âmes multiples définit une section transversale circulaire le long de la deuxième région de détection (108), et dans lequel au moins trois des deuxièmes âmes (150) de fibre optique sont disposées adjacentes à une surface circonférentielle de l'instrument (100) à fibre optique à âmes multiples le long de la deuxième région, et **caractérisé en ce qu'**au moins une des au moins trois deuxièmes âmes (150) de fibre optique est disposée à la même position angulaire qu'une des au moins trois premières âmes (110) de fibre optique.

2. Instrument (100) à fibre optique à âmes multiples selon la revendication 1, dans lequel :
l'ensemble de premiers réseaux réfléchissants (210) est configuré pour fonctionner sur toute l'étendue d'un ensemble de premières largeurs spectrales d'un spectre lumineux de sorte que chaque premier réseau réfléchissant (210) d'une première âme de fibre fonctionne sur toute l'étendue d'une largeur spectrale différente des autres premiers réseaux réfléchissants (210) de la une première âme de fibre, et
l'ensemble de deuxièmes réseaux réfléchissants (250) est configuré pour fonctionner sur toute l'étendue d'un ensemble de deuxièmes largeurs spectrales du spectre lumineux de sorte que chaque deuxième réseau réfléchissant (250) d'une deuxième âme de fibre fonctionne sur toute l'étendue d'une largeur spectrale différente des autres deuxièmes réseaux réfléchissants (250) de la une deuxième âme de fibre,
de préférence dans lequel l'ensemble de premières largeurs spectrales sont les mêmes pour chaque première âme de fibre, et l'ensemble de deuxièmes largeurs spectrales sont les mêmes pour chaque deuxième âme de fibre.

3. Instrument (100) à fibre optique à âmes multiples selon la revendication 2, dans lequel une portion des deuxièmes largeurs spectrales sont un sous-ensemble des premières largeurs spectrales.

4. Instrument (100) à fibre optique à âmes multiples selon l'une ou l'autre de la revendication 2 ou de la revendication 3, dans lequel une majorité de l'ensemble de deuxièmes largeurs spectrales sont un sous-ensemble des premières largeurs spectrales.

5. Instrument (100) à fibre optique à âmes multiples selon l'une quelconque des revendications 2 à 4, dans lequel une totalité substantielle des deuxièmes largeurs spectrales sont un sous-ensemble des premières largeurs spectrales.

6. Instrument (100) à fibre optique à âmes multiples selon l'une quelconque des revendications précédentes, dans lequel :
les premiers réseaux réfléchissants (210) sont configurés pour déterminer un ou plusieurs paramètres physiques de l'instrument (100) à fibre optique à âmes multiples le long de la première région de détection (106), et
les deuxièmes réseaux réfléchissants (250) sont configurés pour déterminer les un ou plusieurs paramètres physiques de l'instrument (100) à fibre optique à âmes multiples le long de la deuxième région de détection (108),
de préférence dans lequel les paramètres physiques comprennent au moins l'un parmi une direction de cintrage, une forme, une contrainte de torsion, une contrainte longitudinale et un mouvement de l'instrument (100) à fibre optique à âmes multiples.

7. Instrument (100) à fibre optique à âmes multiples selon l'une quelconque des revendications précédentes,
dans lequel les au moins trois premières âmes (110) de fibre optique sont espacées de manière égale le long de la surface circonférentielle,
de préférence dans lequel les au moins trois premières âmes (110) de fibre optique s'étendent le long de la première région à une position angulaire constante par rapport à l'instrument.

8. Instrument (100) à fibre optique à âmes multiples selon l'une quelconque des revendications précédentes, dans lequel les au moins trois deuxièmes âmes (150) de fibre optique sont espacées de manière égale le long de la surface circonférentielle de la deuxième région,
de préférence dans lequel les au moins trois deuxièmes âmes (150) de fibre optique s'étendent le long de la deuxième région de détection (108) à une position angulaire constante par rapport à l'instrument (100) à fibre optique à âmes multiples.

9. Instrument (100) à fibre optique à âmes multiples selon la revendication 8, dans lequel chacune des au moins trois deuxièmes âmes (150) de fibre optique est disposée à la même position angulaire qu'une distincte des au moins trois premières âmes (110) de fibre optique.

10. Instrument (100) à fibre optique à âmes multiples selon l'une quelconque des revendications précédentes, dans lequel la deuxième région de détection (108) chevauche au moins partiellement la première région de détection (106),
de préférence dans lequel au moins une des premières âmes (110) de fibre optique est disposée adjacente à la surface circonférentielle le long de la deuxième région de détection (108).

11. Instrument (100) à fibre optique à âmes multiples selon l'une quelconque des revendications précédentes,
dans lequel l'instrument (100) à fibre optique à âmes multiples est incorporé dans l'un parmi un fil introducteur, un fil-guide, un stylet, une sonde, une gaine, un treillis, un tube, un obturateur, ou un cathéter.

12. Système médical pour détecter un ou plusieurs paramètres expérimentés par un dispositif médical allongé inséré à l'intérieur d'un corps de patient, le système comprenant :
l'instrument (100) à fibre optique à âmes multiples allongé selon l'une quelconque des revendications précédentes, dans lequel :
l'instrument (100) à fibre optique à âmes multiples est incorporé dans le dispositif médical allongé, et
le dispositif médical est l'un parmi un fil introducteur, un fil-guide, un stylet, une sonde, une gaine, un treillis, un tube, un obturateur, ou un cathéter ; et
une console (310) incluant une logique optique (380) incluant une source lumineuse (382) et un récepteur optique (384), un ou plusieurs processeurs (360) et un support non transitoire lisible par ordinateur sur lequel est stockée une logique qui,
lorsqu'elle est exécutée par les un ou plusieurs processeurs, provoque des opérations incluant : la fourniture d'un signal lumineux incident à l'instrument (100) à fibre optique à âmes multiples,
la réception de signaux lumineux réfléchis par les premiers réseaux réfléchissants (210) sur toute l'étendue d'un ensemble de premières largeurs spectrales,
la réception de signaux lumineux réfléchis par les deuxièmes réseaux réfléchissants (250) sur toute l'étendue d'un ensemble de deuxièmes largeurs spectrales, les deuxièmes largeurs spectrales étant un sous-ensemble des premières largeurs spectrales,
le traitement des signaux lumineux réfléchis associés aux premiers réseaux réfléchissants (210) et aux deuxièmes réseaux réfléchissants (250),
la détermination d'une forme de l'instrument (100) à fibre optique à âmes multiples le long de la première région de détection (106) à partir de la lumière réfléchie associée aux premiers réseaux réfléchissants (210),
la détermination d'une forme de l'instrument (100) à fibre optique à âmes multiples le long de la deuxième région de détection (108) à partir de la lumière réfléchie associée aux deuxièmes réseaux réfléchissants (250), et
le fait de provoquer un rendu sur un affichage graphique de la forme de l'instrument (100) à fibre optique à âmes multiples le long de la première région de détection (106) et de la deuxième région de détection (108).

13. Système selon la revendication 12, dans lequel l'instrument (100) à fibre optique à âmes multiples comprend en outre un ensemble de troisièmes âmes (170) de fibre optique s'étendant le long de l'instrument (100) à fibre optique à âmes multiples, chaque troisième âme (170) de fibre optique comprenant au moins un troisième capteur, et dans lequel la logique, lorsqu'elle est exécutée par les un ou plusieurs processeurs (360), provoque en outre des opérations incluant :
la réception de signaux lumineux réfléchis par l'au moins un troisième capteur sur toute l'étendue d'une première largeur spectrale,
la détermination d'un paramètre physiologique du corps de patient à partir de la lumière réfléchie associée à l'au moins un troisième capteur, et
le fait de provoquer un rendu sur l'affichage graphique représentant le paramètre physiologique le long de l'instrument (100) à fibre optique à âmes multiples en combinaison avec la forme de l'instrument (100) à fibre optique à âmes multiples le long de la première région de détection (106) et de la deuxième région de détection (108).

14. Système selon la revendication 13, dans lequel le paramètre physiologique est l'un parmi une température corporelle, un débit sanguin ou une oxymétrie de pouls.
